# EUROPÄISCHE PATENTANMELDUNG

(11) **EP 2 460 574 A1**
(43) Veröffentlichungstag der Anmeldung: **06.06.2012**
(21) Anmeldenummer: 11401651.2
(22) Anmeldetag: 01.12.2011
(51) Int. Cl.: A63H 19/36

(54) **Transportable Modellbahn**

(30) Priorität: 02.12.2010 DE 2020100130 U
(71) Anmelder: Dietrich, Frank, 40476 Düsseldorf (DE)
(72) Erfinder: Dietrich, Frank, 40476 Düsseldorf (DE)
(74) Vertreter: Sroka, Peter-Christian

(57) **Zusammenfassung**

Eine transportable Modellbahn, insbesondere Modelleisenbahn oder Modellautobahn, mit mindestens einem Gleis- oder Bahnstrang, der in mindestens zwei Gleis- oder Bahnstrangabschnitte unterteilt ist, von denen jeder auf einer Trägerplatte installiert ist, wobei benachbarte Trägerplatten um eine gemeinsame Scharnierachse relativ zueinander verschwenkbar sind, wobei im Bereich jeder Scharnierachse elektrische Kontaktelemente vorgesehen sind, um im auseinandergeklappten Zustand der Trägerplatten die Gleis- bzw. Bahnstrangabschnitte elektrisch leitend aneinander anzuschließen, deren außen liegende Schienen bzw. Bahnen durch an den Trägerplatten angeordnete Wandstreifen nach außen hin abgeschirmt sind, ist dadurch gekennzeichnet, dass auch die innen liegenden Bahnen bzw. Schienen durch an den Trägerplatten angeordnete Wandstreifen (2.1) nach innen hin abgeschirmt sind.

## Beschreibung

Die Erfindung betrifft eine transportable Modellbahn gemäß dem Oberbegriff des Patentanspruchs 1.

Eine in der DE 809 399 beschriebene transportable Modelleisenbahn enthält nach Art eines Koffers aufklappbare Kastenhälften, an deren Deckeln und Böden innen die Gleisstränge und sonstige Modelleisenbahn-Elemente befestigt sind. Die Gleisstränge sind nach außen hin durch Randleisten abgeschirmt.

Weitere Modelleisenbahnanlagen, bei denen die Gleis- oder Bahnstränge auf zusammenklappbaren Trägerplatten oder dergleichen befestigt sind, sind beschrieben in der DE 19 85 944, der DE 43 16 006 A1 und den US-PS'en 2,616,630 und 2,618,437.

Im Bereich der Scharnierachse sind elektrische Kontaktselemente zum elektrischen Anschließen bzw. Verbinden der Gleisabschnitte vorgesehen.

Der Erfindung liegt die Aufgabe zugrunde, eine derartige Modelleisenbahnanlage so zu gestalten, dass sie ohne Beeinträchtigung der Gleis- oder Bahnstrangabschnitte auch zum Transport sonstiger Gegenstände geeignet ist.

Zu diesem Zweck sind erfindungsgemäß die innen liegenden Bahnen bzw. Gleisschienen durch an den Trägerplatten angeordnete Wandstreifen nach innen hin abgeschirmt. Dadurch wird ohne Beeinträchtigung der eigentlichen Modellbahnanlage ein zusätzlicher Koffer-Stauraum gebildet.

Gemäß einer bevorzugten Ausführungsform der Erfindung ist jeweils ein Gleisabschnitt entlang der Innenseite jeder Koffergriffhälfte angeordnet.

Eine weitere bevorzugten Ausführungsform der Erfindung ist dadurch gekennzeichnet, dass die Trägerplatten im wesentlichen die Form von relativ zueinander verschwenkbaren streifenförmigen Rahmenhälften haben.

Weitere bevorzugte Ausführungsformen der Erfindung sind in Unteransprüchen behandelt.
Figur 1 ist eine perspektivische Außenansicht eines ein Modelleisenbahn-Gleissystems aufnehmenden, geschlossenen Koffers;
Figur 2 zeigt den Koffer in teilweise geöffnetem Zustand;
Figur 3 zeigt einen ein Modelleisenbahn-Gleissystem aufnehmenden Klapprahmen;
Figur 4 zeigt den Klapprahmen im auseinandergeklappten Zustand;
Figur 5 zeigt Kontaktelemente im Bereich des Klappscharniers eines Gleisstrangs;
Figur 6 zeigt zwei sich gegenüberliegende und mittels Kontaktelementen elektrisch aneinander anschließbare Gleisstränge im Bereich des Klappscharniers;
Figuren 7 und 8 zeigen eine abgewandelte Ausführungsform von Kontaktelementen.

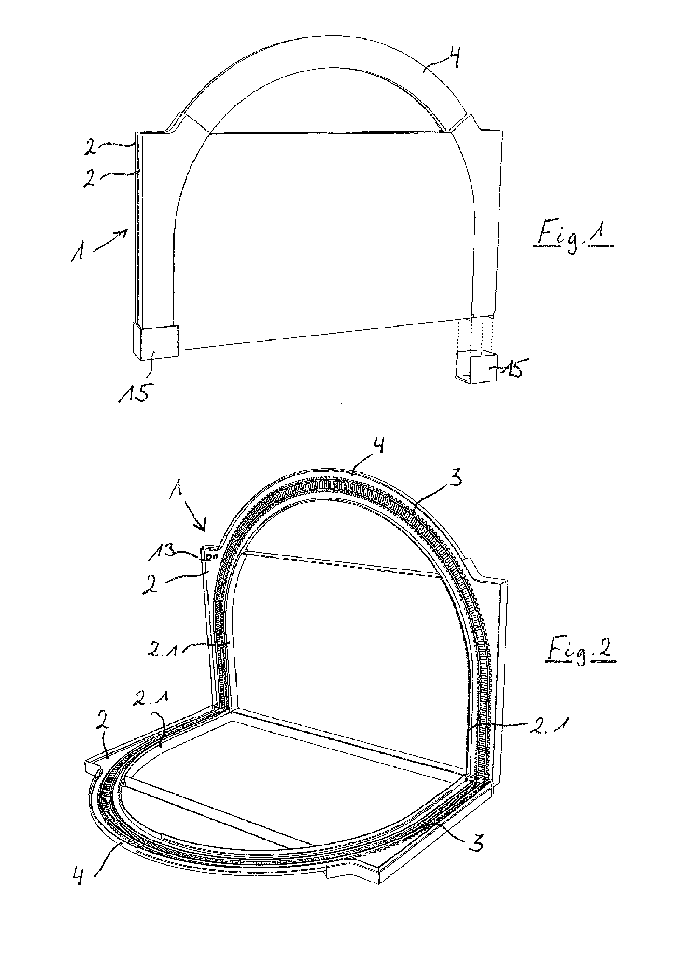

Gemäß Figur 2 sind die Gleisstränge 3 auf Trägerplatten angeordnet, die den Boden bzw. den Deckel eines Koffers 1 bilden.

Die Figuren 1 und 2 zeigen einen Koffer 1 mit zwei auseinander- und zusammenklappbaren Kofferhälften 2, die jeweils einen bogenförmigen Gleisstrang 3 aufnehmen, wobei ein Abschnitt jedes Gleisstranges 3 entlang der Innenseiten der Koffergriffe 4 verläuft. In dem Kofferinnenraum sind die Gleisstrangabschnitte vorzugsweise nach Innen hin abgeschirmt, beispielsweise mittels Wandstreifen 2.1. Die bei geschlossenem Koffer sich gegenüberliegenden Gleisstrangabschnitte bzw. die im Folgenden noch zu beschreibenden elektrischen Anschluss- bzw. Verbindungselemente, insbesondere elektrischen Kontaktelemente sich gegenüberliegender Gleisstrangabschnitte sind bei geschlossenem Koffer vorzugsweise durch aufschiebbare Gleitschutzkappen 15 geschützt.

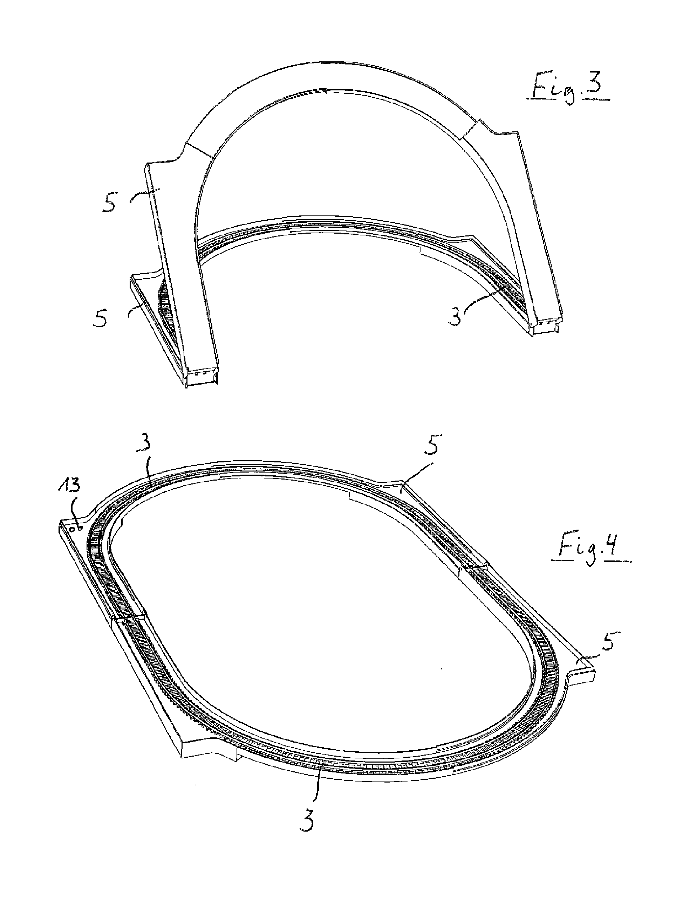

Bei der Ausführungsform gemäß der Figuren 3 und 4 sind die bogenförmigen Gleisstrangabschnitte 3 an der Innenseite von zwei auseinander- und zusammenklappbaren Trägerplatten im wesentlichen in Form von streifenförmigen Rahmenhälften angeordnet.

Zum Anschließen der Gleise an eine Stromquelle können handelsübliche elektrische Anschluss- bzw. Verbindungselemente, z.B. Kontaktzungen, elektrische Leitung oder Kabel oder andere geeignete Einrichtungen, verwendet werden. Gemäß der Zeichnung können z.B. Steckerbuchsen 13 zum Einstecken von z.B. Eisenbahnsteckern vorgesehen sein.

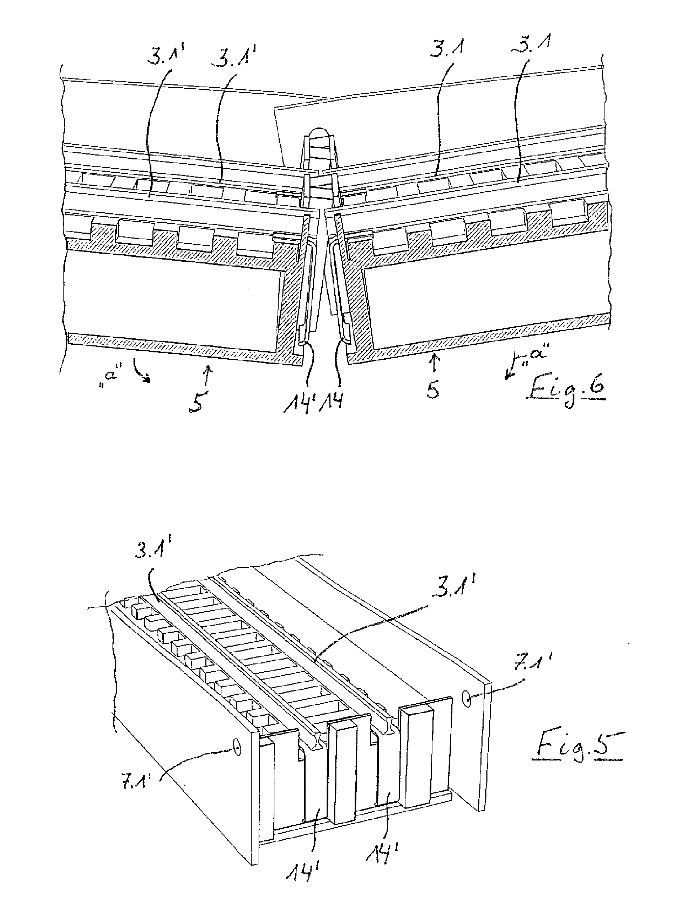

Gemäß Figur 5 steht im Bereich des durch eine Öffnung 7.1' repräsentierten Scharniers jedes Ende einer Schiene 3.1' in elektrisch leitendem Kontakt mit einer Kontaktzunge 14, die in Gleisstrangrichtung nach außen federnd vorgespannt ist. Wenn die beiden in Figur 6 dargestellten Rahmenhälften 5 in Richtung der Pfeile "a" weiter verschwenkt werden, kommen die sich gegenüberliegenden Kontaktzungen 14, 14' in elektrischen Kontakt miteinander, so dass im Bereich des Scharniers die sich gegenüberliegenden Schienen 3.1; 3.1' elektrisch aneinander angeschlossen, d.h. elektrisch miteinander verbunden sind.

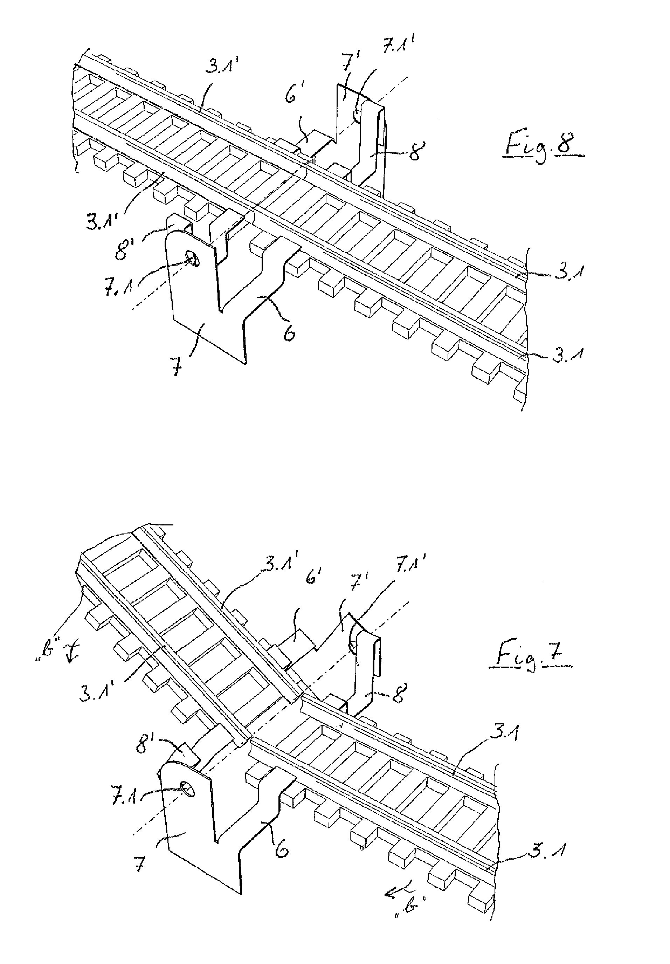

Bei der Ausführungsform gemäß den Figuren 7 und 8 sind seitlich neben den Schienen 3.1, 3.1' angeordnete Kontaktelemente vorgesehen, um im auseinandergeklappten Zustand des Koffers 1 bzw. des Rahmens 5 die Schienen 3.1; 3.1' elektrisch leitend aneinander anzuschließen.

Gemäß Figur 7 ist an eine Schiene 3.1 ein elektrisch leitender Kontaktstreifen 6 angeschlossen, der im Wesentlichen senkrecht zur Schiene 3.1 ausgerichtet ist und zu einem parallel zur Schiene 3.1 ausgerichteten Kontaktblech 7 führt, das mit einer das Scharnier repräsentierenden Öffnung 7.1 versehen ist. An die gegenüberliegende Schiene 3.1 ist eine elektrisch leitende Kontaktzunge 8 angeschlossen, die relativ zur Schiene 3.1 federnd nach außen, d.h. weg von der Schiene 3.1 vorgespannt ist.

Den Schienen 3.1' sind zu den Kontaktelementen 6, 7 und 8 diagonal spiegelbildlich identische Kontaktelemente 6', 7' bzw. 8' zugeordnet. Beim Verschwenken der beiden Kofferhälften in Richtung der Pfeile "b" gleiten die nach außen vorgespannten Kontaktzungen 8 bzw. 8' entlang der Innenflächen der beiden Kontaktflächen 7 bzw. 7' derart, dass in dem in Figur 8 dargestellten auseinandergeklappten Zustand die dann miteinander fluchtenden Schienen 3.1; 3.1' elektrisch leitend aneinander angeschlossen sind.

## Patentansprüche

1. Transportable Modellbahn, insbesondere Modelleisenbahn oder Modellautobahn, mit mindestens einem Gleis- oder Bahnstrang (3), der in mindestens zwei Gleis- oder Bahnstrangabschnitte unterteilt ist, von denen jeder auf einer Trägerplatte installiert ist, wobei benachbarte Trägerplatten um eine gemeinsame Scharnierachse relativ zueinander verschwenkbar sind, wobei im Bereich der Scharnierachse angeordnete elektrisch leitende Kontaktelemente (14, 14' bzw. 6, 7, 8, 6', 7' 8') vorgesehen sind, um bei auseinandergeklappten Trägerplatten die Gleis- bzw. Bahnstrangabschnitte elektrisch leitend aneinander anzuschließen, deren außen liegende Schienen bzw. Bahnen durch an den Trägerplatten angeordnete Wandstreifen nach außen hin abgeschirmt sind, **dadurch gekennzeichnet, dass** auch die innen liegenden Bahnen bzw. Schienen (3.1) durch an den Trägerplatten angeordnete Wandstreifen (2.1) nach innen hin abgeschirmt sind.

2. Transportable Modellbahn nach Anspruch 1, **dadurch gekennzeichnet, dass** die Trägerplatten die Boden- bzw. Deckelflächen von kastenförmigen Kofferhälften (4) bilden, von denen jede mit einer Koffergriffhälfte versehen ist, an deren Innenseiten jeweils ein Abschnitt eines Bahn- bzw. Gleitstranges (3) angeordnet ist.

3. Transportable Modellbahn nach Anspruch 1, **dadurch gekennzeichnet, dass** die Trägerplatten im wesentlichen die Form von relativ zueinander verschwenkbaren streifenförmigen Rahmenhälften haben.

4. Transportable Modellbahn nach Anspruch 1, **dadurch gekennzeichnet, dass** jedes Ende einer Schiene (3.1; 3.1') oder Bahn in elektrisch leitendem Kontakt mit einer Kontaktzunge (14; 14') steht, die in Gleis- oder Bahnrichtung nach außen federnd vorgespannt ist, derart, dass bei auseinander geklappten Trägerplatten die sich gegenüberliegenden Kontaktzungen (14; 14') in elektrischen Kontakt kommen.

5. Transportable Modellbahn nach Anspruch 4, **dadurch gekennzeichnet, dass** seitlich neben den Schienen (3.1; 3.1') bzw. Bahnen mit den Schienen (3.1; 3.1') bzw. Bahnen in Kontakt stehende Kontaktelemente vorgesehen sind, die in auseinander geklappten Zustand des Koffers (1) bzw. des Rahmens (5) die Enden der Schienen (3.1; 3.1') bzw. Bahnen elektrisch leitend aneinander anschließen bzw. miteinander verbinden.

6. Transportable Modellbahn nach Anspruch 5, **dadurch gekennzeichnet, dass** an eine Schiene (3.1) bzw. Bahn ein elektrisch leitender Kontaktstreifen (6) angeschlossen ist, der im wesentlichen senkrecht zur Schiene (3.1) bzw. Bahn ausgerichtet ist und zu einem parallel zur Schiene (3.1) bzw. Bahn ausgerichteten Kontaktblech (7) führt, und dass an die gegenüberliegende Schiene (3.1) bzw. Bahn eine elektrisch leitende Kontaktzunge (8) angeschlossen ist, die relativ zur Schiene (3.1) bzw. Bahn federnd nach außen, d.h. weg von der Schiene (3.1) bzw. Bahn vorgespannt ist, und dass den Schienen (3.1') bzw. Bahnen diagonal spiegelbildlich zu den Kontaktelementen (6, 7 und 8) Kontaktelemente (6', 7' bzw. 8') zugeordnet ist, derart, dass beim Auseinanderklappen der Koffer- bzw. Rahmenhälften die nach außen vorgespannten Kontaktzungen (8 bzw. 8') entlang der Innenflächen der beiden Kontaktflachen (7 bzw. 7') gleiten, derart, dass im auseinander geklappten Zustand der Rahmen- bzw. Kofferhälften die dann miteinander fluchtenden Schienen (3.1; 3.1') bzw.

7. Transportable Modellbahn nach einem der Ansprüche 1 bis 6, **gekennzeichnet durch** in mindestens einer der beiden Rahmen- bzw. Kofferhälften angeordnete Steckerbuchsen (13) zum Einstecken von Eisenbahnsteckern, die an eine Stromversorgungsquelle angeschlossen sind.

8. Transportable Modellbahn nach Anspruch 1, **dadurch gekennzeichnet, dass** bei auseinander geklappten Trägerplatten die sich gegenüberliegenden Schienen bzw. Bahnen mittels elektrischer Kabel miteinander verbunden sind.

9. Transportable Modelleisenbahn nach einem der Ansprüche 1 bis 8, **gekennzeichnet durch** Schutzkappen (15), die im zusammengeklappten Zustand der Trägerplatten im Bereich der Kontaktelemente auf die Trägerplatten aufschiebbar sind.
